# EUROPEAN PATENT SPECIFICATION

(11) **EP 4 227 290 B1**
(45) Date of publication and mention of the grant of the patent: **22.05.2024**
(21) Application number: 20958334.3
(22) Date of filing: 23.10.2020
(51) Int. Cl.: C07C 233/03, C07C 233/05, C07C 231/24, C02F 103/36, C02F 1/26

(54) **DMF RECYCLING METHOD**
VERFAHREN ZUR WIEDERVERWENDUNG VON DMTM
PROCÉDÉ DE RECYCLAGE DE DMF

(43) Date of publication of application: 16.08.2023
(73) Proprietor: Anhui Jinhe Industrial Co., Ltd., Chuzhou, Anhui 239200 (CN)
(72) Inventor: ZHAO, Jingang, Chuzhou, Anhui 239200 (CN); SHI, Jian, Chuzhou, Anhui 239200 (CN); CHEN, Yongle, Chuzhou, Anhui 239200 (CN)
(74) Representative: Ipside
(86) International application number: PCT/CN2020/123334
(87) International publication number: WO 2022/082752

(56) References cited:
- WO-A1-2008/095614
- CN-A- 100 999 480
- CN-A- 105 646 271
- CN-A- 106 831 472
- CN-A- 108 862 795
- CN-A- 110 423 192
- CN-A- 111 574 394
- CN-A- 111 646 919
- CN-A- 111 646 919
- CN-A- 112 574 059
- CN-B- 108 358 807

## Description

### Technical Field

The present invention relates to the technical field of chemical product recycling industry, and in particular to a DMF recycling method that can be used in the production process of sucralose.

### Background of the Invention

Sucralose, commonly known as sucralose, is a non-nutritive powerful sweetener with high sweetness, no energy, pure sweetness and high safety, and is the second largest class of food additives after condiments. At present, there are three main methods for synthesizing sucralose, including whole group protection method, single group protection method and combined enzyme-chemical method. Among them, the single group protection method has become the present mainstream technology for the production of sucralose due to its high yield and large production capacity. This method mainly includes esterification, acylation, chlorination, hydrolysis, purification and other processes, wherein sucrose, acetic anhydride, organotin, trichloroethane, dimethylformamide (DMF) and ethyl acetate are used respectively as raw material, catalyst or solvent.

As one of the most used solvents in sucralose production, dimethylformamide (DMF) can effectively reduce the production cost of the process if it can be reused. A recycling method is disclosed for instance in CN111646919A. However, acetic acid chloride ions and water are produced during the production of sucralose, which are entrapped in the DMF solvent, and the process of recycling such DMF mixed solution is often accompanied by serious corrosion of the equipment, and the DMF recycled from such DMF mixed solution has many impurities and poor purity, thus affecting product quality of esterification section, acylation section and chlorination section in the process of reuse, and ultimately affect the production of sucralose. Therefore, it is of great research significance to develop a method to recycle DMF in sucralose production process.

### Summary of the Invention

In view of the lack of purity of DMF recycled from the DMF mixture solution of sucralose industry in the prior art, which affects the quality of sucralose, a DMF recycling method of the present application is proposed in order to overcome the above problem.

In order to achieve the above purpose, the present application employs the following technical solution:
A DMF recycling method, wherein the DMF stock solution (i.e., the mixed solution containing DMF solvent collected in the sucralose production process) used for recycling includes DMF, acetic acid and water, is characterized in that the method comprises the steps:
Neutralization step: adding alkaline neutralizer to the DMF stock solution, controlling pH value of a mixture within a preset range, and performing a neutralization reaction to convert acetic acid in the DMF stock solution into acetate;
Extraction and crystallization step: adding an anti-solvent that is insoluble with the acetate to the neutralized solution, mixing and stirring to extract DMF by the anti-solvent, thereby causing an acetate hydrate to crystallize and precipitate, and filtering the solid to obtain mother liquor containing DMF;
Layering step: standing and layering the mother liquor containing DMF to obtain an upper layer liquid phase and a lower layer liquid phase;
Evaporation step: the upper layer liquid phase and the lower layer liquid phase are evaporated and concentrated separately; a condensate obtained by evaporation of the upper layer liquid phase is recycled into the anti-solvent, a concentrated solution obtained by evaporation of the upper layer liquid phase and a condensate obtained by evaporation of the lower layer liquid phase are mixed to obtain a crude DMF solution for recycling of DMF.

Optionally, in the DMF stock solution: DMF mass content is 75% to 95%, water mass content is 4% to 15%, and acetic acid mass content is 1% to 10%.

Optionally, the alkaline neutralizer is liquid alkali or solid alkali;
The liquid alkali is one of NaOH or KOH aqueous solution, and mass concentration is 1% to 50%;
The solid alkali is one of NaHCOs, Na₂CO₃, KHCOs, K₂CO₃ or Ca(OH)₂, or a mixture of NaHCOs and Na₂CO₃, or a mixture of KHCOs and K₂CO₃, preferably Ca(OH)₂.

Optionally, the amount of solute in the liquid alkali is 0.8 to 1 times of the amount of substance of acetic acid, reaction temperature is room temperature, reaction time is 5 min to 15 min, and stirring rate is 100 r/min to 300 r/min.

Optionally, the amount of the solid alkali is 1 to 3 times of the amount of substance of acetic acid, reaction temperature is 40°C to 70°C, reaction time is 0.5h to 1.5h, and stirring rate is 200 r/min to 400 r/min.

Optionally, in the neutralization step, the pH of the mixture is controlled from 7 to 11 by adding an alkaline neutralizing agent.

Optionally, the anti-solvent is one of ether, anisole, isopropyl ether, benzene, toluene and xylene; preferably the anti-solvent is toluene.

Optionally, the anti-solvent is added in the amount of: volume ratio of DMF stock solution: anti-solvent = 1: 0.5 to 5; preferably: volume ratio of DMF stock solution: anti-solvent = 1: 1 to 3.

Optionally, in the extraction and crystallization step, the temperature of anti-solvent extraction and crystallization process is room temperature, the stirring rate is 200 r/min to 400 r/min and the stirring time is 15 min to 60min.

Optionally, the method also comprises the steps:
Adjustment step: adding an acidic regulator to the crude DMF solution to adjust the pH of the crude DMF solution to 6-8, preferably 7, and the adjusted crude DMF solution is used to recycle DMF.

Optionally, the acid regulator used in the adjustment step is inorganic acid, preferably a mixture of one or more of sulfuric acid, phosphoric acid solution, with a mass concentration of 5% to 50%.

Optionally, the method also comprises the steps:
Secondary recycling step: recycling acetate hydrate solid precipitated in the extraction and crystallization step and/or acetate hydrate solid obtained by evaporation of the lower layer liquid phase in the evaporation step.

In summary, the beneficial effect of the present application is:
The present application converts the acetic acid in the DMF stock solution into acetate by adding an alkaline neutralizer, followed by extraction and crystallization by adding an anti-solvent that is insoluble with the acetate to achieve the removal of acetic acid, and then obtains a crude DMF solution almost free of acetic acid by layering and evaporation of the mother liquor, and using this crude DMF solution for DMF recycling can improve the purity of the recycled DMF and avoid tetramethylurea produced by the reaction with metal equipment due to the presence of the acetic acid, thus ultimately improve the quality of sucralose.

### Brief description of the Drawings

FIG. 1 is a step flow diagram of a DMF recycling method provided in an embodiment of the present application;
FIG. 2 a process flow diagram of a DMF recycling method provided in an embodiment of the present application;

In the figures: E-1 is a neutralization kettle, E-2 is a filter, E-3 is a mixing kettle, E-4 is a solid separator, E-5 is a liquid phase separator, E-6 is an evaporator 1, E-7 is an evaporator 2, and E-8 is a pH adjustment tank .

### Detailed Description of Embodiments

In order to make the purpose, technical solutions and advantages of the application clearer, the implementation of the application will be further described in detail below in conjunction with the accompanying drawings.

In the description of the application, it should be noted that orientation or positional relationship indicated by terms "center", "upper", "lower", "left", "right", "vertical", "horizontal", "inner", "outside" etc. is based on orientation or positional relationship shown in the drawings, which is only for convenience of describing the application and simplifies the description, rather than indicates or implies that the device or element referred to must have a particular orientation, be constructed and operated in a particular orientation, and therefore are not to be construed as limiting the application. In addition, the terms "first", "second", and "third" are used for descriptive purposes only and are not to be understood as indicating or implying relative importance.

In the description of the application, it should be noted that, unless otherwise specified and limited, terms "installation", "connected" and "connection" should be understood in a broad sense, for example, it can be a fixed connection, a removable connection, or a one-piece connection; a mechanical connection or an electrical connection; a direct connection or an indirect connection through an intermediate medium, or a connection within two components. For a person of ordinary skill in the art, the specific meaning of the above terms in the context of the present application can be understood in specific situation.

By studying the DMF stock solution produced in the sucralose production process (i.e., the mixed solution containing DMF solvent collected in the sucralose production process), the applicant found the following problem: since sucralose produces acetic acid, chloride ions and water during the production process, which are entrapped in the DMF solvent, causing the DMF mixed solvent to be acidic, and as the recycling step continues, the acetic acid and chloride ion concentration will continue to rise, thus causing serious equipment corrosion.

The specific principle is: when there are chloride ions in the DMF stock solution system, the chloride ions will destroy the oxidation layer on the metal surface, thus exposing the internal metal outside, and as everyone knows, removal of chloride ions is difficult and expensive in water-soluble systems, which is a hard problem in the chemical industry; when there are hydrogen ions in the system, they will react with the metal, thus causing surface corrosion of metal products, while acetic acid is the main source of hydrogen ions in DMF solution. In addition, when stainless steel is selected, especially 316L (a steel material grade), the nickel metal in this material will form nickel acetate metal organic salts after corrosion, and this type of water-soluble metal salt is a kind of Lewis acid that can be used as an organic reaction catalyst to attack the C=O group on the DMF molecule at high temperatures, activating it and interacting with another DMF molecule to form a tetramethylurea. The boiling point of tetramethylurea is at 177°C, which is higher than the boiling point of DMF at 152.5°C such that it can be remained and enriched in DMF as impurities during the separation process, thus affecting the product quality in the esterification, acylation and chlorination sections during the reuse of DMF.

Thus, for the DMF stock solution containing acetic acid generated in the sucralose production process, if the recycling is performed using traditional methods, the recycling process causes serious corrosion of metal equipment, increasing the equipment maintenance cost, and the recycled DMF solvent is lack of purity, which ultimately affects the quality of sucralose.

For the above problem found, the present application proposes a DMF recycling method, the technical concept of which is: by adding an alkaline neutralizer, the acetic acid in the DMF stock solution is converted into acetate, then by adding an anti-solvent insoluble with acetate, extraction and crystallization is performed to remove the acetic acid, and then the mother liquor is layered and evaporated to obtain a crude DMF solution almost free of acetic acid, and the crude DMF solution is used for DMF recycling, such that the purity of recycled DMF can be improved and the tetramethylurea produced by the reaction with metal equipment due to the presence of acetic acid can be avoided, thus ultimately improving the quality of sucralose.

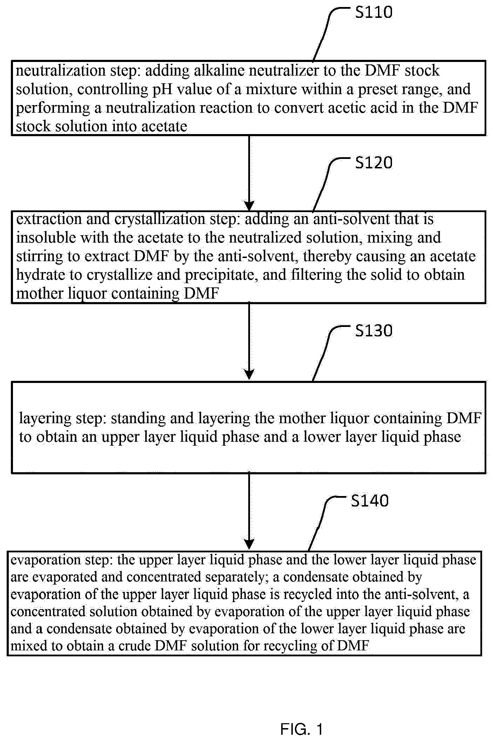

FIG. 1 is a step flow diagram of a DMF recycling method provided in an embodiment of the present application, as shown in FIG. 1, a DMF recycling method in which the DMF stock solution used for recycling comprises DMF, acetic acid and water, wherein the method comprises the steps:
S110 neutralization step: adding alkaline neutralizer to the DMF stock solution, controlling pH value of a mixture within a preset range, and performing a neutralization reaction to convert acetic acid in the DMF stock solution into acetate.
S120 extraction and crystallization step: adding an anti-solvent that is insoluble with the acetate to the neutralized solution, mixing and stirring to extract DMF by the anti-solvent, thereby causing an acetate hydrate to crystallize and precipitate, and filtering the solid to obtain mother liquor containing DMF.
S130 layering step: standing and layering the mother liquor containing DMF to obtain an upper layer liquid phase and a lower layer liquid phase.
S140 evaporation step: the upper layer liquid phase and the lower layer liquid phase are evaporated and concentrated separately; a condensate obtained by evaporation of the upper layer liquid phase is recycled into the anti-solvent, a concentrated solution obtained by evaporation of the upper layer liquid phase and a condensate obtained by evaporation of the lower layer liquid phase are mixed to obtain a crude DMF solution for recycling of DMF.

By the above steps, in the process of recycling DMF of the present application, because it takes the lead in removing acetic acid from the DMF stock solution through the S110 neutralization step and the S120 extraction and crystallization step, the impurity tetramethylurea will not be produced by reacting with the metal equipment due to the presence of acetic acid. Thus, by using the DMF recycling method of the present application, it is possible to reduce the tetramethylurea in the recycled product, thereby recycling DMF from the DMF stock solution with higher purity, and at the same time, it is possible to avoid corrosion of the metal equipment during the recycling process and reduce the maintenance cost of the equipment.

In one embodiment of the present application, the DMF stock solution contains: 75-95% DMF by mass; 4-15% water by mass, and 1-10% acetic acid by mass. Of course, the DMF stock solution may also contain other impurities, such as chloride ions, etc., which are not listed in detail here. Moreover, the above DMF stock solution component is the preferred range of better effect of applying the DMF recycling method of this application, in addition, for DMF stock solution containing different ratios of acetic acid and water, the DMF recycling method of this application can be applied to have different degrees of recycling enhancement effect.

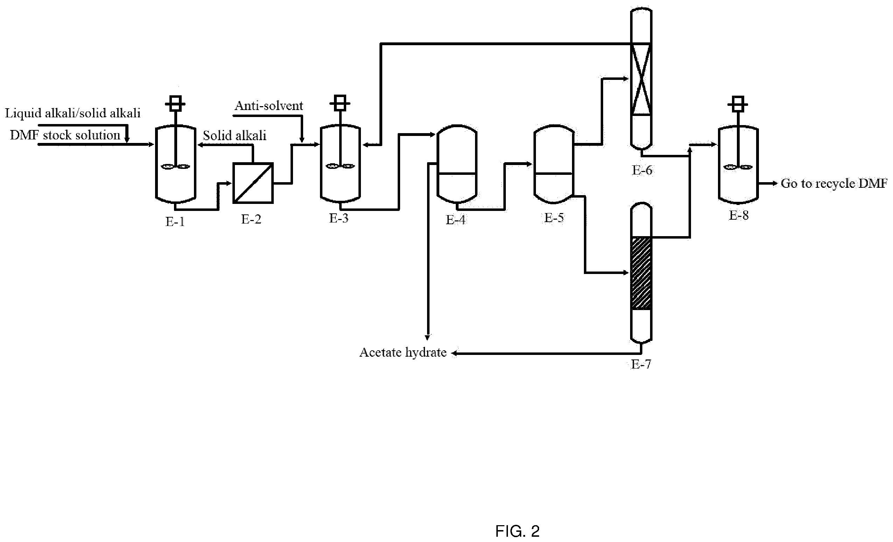

FIG. 2 illustrates a feasible process flow diagram for the DMF recycling method of the present application.

Referring to the process flow diagram of the DMF recycling method shown in FIG. 2, in industrial production, firstly, a certain component of DMF stock solution and an alkaline neutralizing agent to promote the complete reaction of acetic acid in the stock solution are added to the E-1 neutralizing kettle, the alkaline neutralizing agent can be a liquid alkali or a solid alkali, the reaction is done in room temperature or by heating and stirring, after a certain time of reaction, the reaction solution added with the liquid alkali is cooled to room temperature and then enters into the E-3 mixing kettle; the reaction solution added with the solid alkali is cooled to room temperature and then filtered through E-2 filter, the filtered solution enters the mixing kettle E3, and the filtered solid alkali is separated and returned to the neutralizing kettle for reuse.

Next, a certain proportion of anti-solvent is added to the E-3 mixing kettle and stirred thoroughly, the mixture is stirred for a certain time and then filtered in the E-4 solid-liquid separator to collect the solid acetate hydrate; the filtered mother liquor enters the E-5 liquid phase separator for static layering. The upper layer liquid phase enters E-6 evaporator 1 to be evaporated at room temperature and atmospheric pressure or negative pressure, the condensate obtained by evaporation is anti-solvent, the anti-solvent returns to E-3 mixing kettle to continue anti-solvent extraction and crystallization, the DMF concentrate collected after evaporation enters E-8 pH adjustment kettle. The lower layer liquid phase enters E-7 evaporator 2 to be evaporated at high temperature and negative pressure, the condensate obtained by evaporation enters E-8 pH adjustment kettle, the solid obtained by evaporation of the lower layer liquid phase is acetate hydrate. The material in E-8 pH adjustment kettle is the crude DMF solution, which goes to recycle DMF after pH adjustment.

In one embodiment of the present application, the alkaline neutralizing agent in the S110 neutralization step is a liquid alkali or a solid alkali. Compared to using a liquid alkali, when using a solid alkali, it is necessary to provide a filter to filter out the solid alkali which is not dissolved and consumed.

The liquid alkali is for example one of an aqueous solution of NaOH or KOH at a concentration of 1 to 50% by mass.

The solid alkali is for example one of NaHCOs, Na₂CO₃, KHCOs, K₂CO₃ or Ca (OH) ₂, or NaHCOs and Na₂CO₃ mixture, or KHCOs and K₂CO₃ mixture, preferably Ca (OH) ₂.

In an embodiment of the present application, the amount of solute in the liquid alkali is 0.8 to 1 times of the amount of the substance of acetic acid, the reaction temperature is room temperature, the reaction time is 5 to 15 min, and the stirring rate is 100 to 300r/min.

In an embodiment of the present application, the amount of solid alkali is 1 to 3 times of the amount of substance of acetic acid, the reaction temperature is 40 to 70°C, the reaction time is 0.5 to 1.5h, and the stirring rate is 200 to 400r/min.

In the neutralization process, the liquid alkali has a faster reaction time due to sufficient contact, and thus has a shorter reaction time. Regardless of whether it is a liquid alkali or a solid alkali, the dosage should be determined by the goal of consuming the acetic acid as completely as possible.

In an embodiment of the present application, in the S110 neutralization step, the pH of the mixture is controlled from 7 to 11 by adding an alkaline neutralizer. By controlling the pH of the mixture from neutral to alkaline to ensure that the acetic acid is completely reacted.

In one embodiment of the present application, the anti-solvent is one of ether, anisole, isopropyl ether, benzene, toluene, and xylene; preferably toluene is the anti-solvent. The anti-solvent is insoluble with acetate; the precipitation of acetate hydrate can be promoted by extraction and crystallization, followed by a simple solid-liquid separation to achieve the separation of acetate in DMF stock solution. The characteristics of the anti-solvent selected in this application are: 1. miscible with DMF, and the boiling point difference is large, thus easy to separate; 2. insoluble with acetate, easier to promote the precipitation of acetate hydrate, to achieve removal of acetic acid; 3. does not react with any component in the system, and does not generate new impurities; 4. stable in nature, and does not decompose due to changes in conditions.

In an embodiment of the present application, the addition amount of the anti-solvent is: volume ratio DMF stock solution: anti-solvent = 1: 0.5 to 5; preferably: volume ratio DMF stock solution: anti-solvent = 1: 1 to 3.

In an embodiment of the present application, the temperature of the anti-solvent extraction and crystallization process in the extraction and crystallization step is room temperature, the stirring rate is 200 to 400 r/min, and the stirring time is 15 to 60 min. Stirring can accelerate the speed of anti-solvent extraction and crystallization to achieve sufficient contact extraction, and promote precipitation of the acetate hydrate due to insolubility with the anti-solvent.

In an embodiment of the present application, the method further comprises the following step:
Adjustment step: adding an acidic regulator to the crude DMF solution to adjust the pH of the crude DMF solution to 6-8, preferably 7. The adjusted crude DMF solution is used to recycle DMF. Adjusting the pH is mainly used to consume the excess alkali in the solution to avoid affecting the subsequent recycling step.

In one embodiment of the application, the acid regulator used in the adjustment step is an inorganic acid, preferably one or more of sulfuric acid and phosphoric acid solution, and the mass concentration is 5-50%. The crude DMF solution after adjusting the pH value can be purified and recycled by rectification. The rectification method is not limited to atmospheric rectification and reduced pressure distillation, etc., in order to obtain high-purity DMF solvent and realize the recycling of DMF.

In an embodiment of the present application, the method further comprises the following step:
A secondary recycling step: recycling acetate hydrate solid precipitated in the extraction and crystallization step and/or acetate hydrate solid obtained by evaporating the lower layer liquid phase in the evaporation step. Acetate hydrate solid can be used as a carbon source for the biochemical system of the municipal wastewater station to feed bacteria, resulting in lower municipal wastewater treatment costs, thus realizing the economic benefits in terms of secondary utilization of the recycled by-products and environmental protection, and further expanding the overall recycling benefit of the DMF recycling method of the present invention.

That is, the present invention is not only capable of recycling high purity DMF solution from DMF stock solution, effectively avoiding corrosion of metal equipment and high maintenance costs due to the presence of impurities such as acetic acid, but also capable of improving the quality of sucralose products, and obtaining acetate hydrate through the secondary recycling step, further increasing the recycling yield while realizing economic benefits in terms of environmental protection.

### Embodiment 1

DMF stock solution (DMF stock solution is an acidic aqueous solution of DMF produced in the esterification section of the sucralose production line. The DMF and acetic acid content is obtained by the quantitative detection of gas chromatography detector, and the water content is obtained by the detection of moisture meter, and the other embodiments are the same) components are shown in Table 1.

**Table 1 DMF stock solution component**

| Component | DMF | water | acetic acid |
|---|---|---|---|
| mass content % | 86.36 | 9.51 | 4.13 |

Step (1): add 200L of DMF stock solution into the neutralization kettle, stir and drip 10.93kg of NaOH aqueous solution (configured from commercially available solid NaOH of 99% purity) with mass fraction of 50%, pH = 11.0 after drop, stir for 10 min at room temperature at a stirring rate of 100r/min, and then input into the mixing kettle.

Step (2): add 600L of ether (99% purity, commercially available) into the mixing kettle, stir rapidly for 1 h at a stirring rate of 300r/min, filter in the solid-liquid separator to obtain 16.03kg of solid sodium acetate trihydrate, the filtered mother liquor enters into the liquid phase separator for static layering, the upper layer enters into evaporator 1 and is evaporated to obtain the condensate ether, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 2.96kg of solid sodium acetate trihydrate; add 30% dilute sulfuric acid (configured by 98% concentrated sulfuric acid) into the pH adjustment kettle to neutralize to pH = 8.0, then the material liquid goes to the DMF recycling system, the component in the pH adjustment kettle is shown in Table 2.

**Table 2 material liquid component to DMF recycling system**

| Component | DMF | water | acetic acid |
|---|---|---|---|
| mass content % | 88.79 | 11.2 | 0.01 |

As can be seen from Table 2, after treatment of the present embodiment, the acetic acid content in the DMF solution is extremely low, so that in the subsequent separation and recycling of DMF solvent process, metal equipment will not react due to the presence of acetic acid, the equipment will not be corroded, and it is generated that no tetramethylurea to be mixed into the DMF, thus, not only to maintain the safety of equipment, but also to improve the recycling purity of DMF. In the following embodiments, the purpose of removing acetic acid and improving the purity of DMF recycling can be achieved, and the content of acetic acid is reduced to less than 0.15%.

### Embodiment 2

DMF stock solution component is shown in Table 3.

**Table 3 DMF stock solution component**

| Component | DMF | water | acetic acid |
|---|---|---|---|
| mass content% | 79.8 | 15 | 5.2 |

Step (1): add 1 m³ DMF stock solution into the neutralization kettle, add 47.51kg of solid Na₂CO₃ (99% purity, commercially available), pH = 9.5 after addition, heat to 50°C, continue to stir and react for 1 h at a stirring rate of 250 r/min, input the filtered mother liquor into the mixing kettle.

Step (2): add 2.75m³ anisole (99% purity, commercially available) into the mixing kettle, stir rapidly for 50 min at a stirring rate of 300 r/min, filter in the solid-liquid separator to obtain 93.92 kg of solid sodium acetate trihydrate, the filtered mother liquor enters into the liquid phase separator for static layering, the upper layer enters into evaporator 1 and is evaporated to obtain the condensate anisole, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 23.95 kg of solid sodium acetate trihydrate; add 20% dilute sulfuric acid (configured by 98% concentrated sulfuric acid) into the pH adjustment kettle to neutralize to pH = 7.5, then the material liquid goes to the DMF recycling system, the component in the pH adjustment kettle is shown in Table 4.

**Table 4 component of the material liquid to DMF recycling system**

| Component | DMF | water | acetic acid |
|---|---|---|---|
| mass content% | 81.65 | 18.2 | 0.15 |

### Embodiment 3

DMF stock solution component is shown in Table 5.

**Table 5 DMF stock solution component**

| Component | DMF | water | acetic acid |
|---|---|---|---|
| mass content % | 85.72 | 4.27 | 10.01 |

Step (1): add 2 m³ DMF stock solution into the neutralization kettle, add 404.01 kg of solid Na₂CO₃ (99% purity, commercially available), pH = 7.5 after addition, heat to 70°C, continue to stir and react for 30 min at a stirring rate of 300 r/min, input the filtered mother liquor into the mixing kettle.

Step (2): add 1 m³ isopropyl ether (99% purity, commercially available) into the mixing kettle, stir rapidly for 15 min at a stirring rate of 200 r/min, filter in the solid-liquid separator to obtain 439.03 kg of solid sodium acetate trihydrate, the filtered mother liquor enters into the liquid phase separator for static layering, the upper layer enters into evaporator 1 and is evaporated to obtain the condensate isopropyl ether, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 12.11 kg of solid sodium acetate trihydrate; add 45% dilute phosphoric acid (configured by 98% concentrated phosphoric acid) into the pH adjustment kettle to neutralize to pH = 7.0, then the material liquid goes to the DMF recycling system, the component in the pH adjustment kettle is shown in Table 6.

**Table 6 component of the material liquid to DMF recycling system**

| Component | DMF | water | acetic acid |
|---|---|---|---|
| mass content% | 96.18 | 3.80 | 0.02 |

### Embodiment 4

DMF stock solution component is shown in Table 7.

**Table 7 DMF stock solution component**

| Component | DMF | water | acetic acid |
|---|---|---|---|
| mass content% | 87.60 | 7.49 | 4.91 |

Step (1): add 1.5 m³ DMF stock solution into the neutralization kettle, stir and drip 91.54 kg of KOH aqueous solution with a mass fraction of 30% (configured from commercially available solid KOH of 99% purity), pH = 9.7 after drop, continue to stir for 5min at a stirring rate of 100 r/min, input it into the mixing kettle.

Step (2): add 1.5 m³ Benzene (99% purity, commercially available) into the mixing kettle, stir rapidly for 20 min at a stirring rate of 200 r/min, filter in the solid-liquid separator to obtain 112.29 kg of solid potassium acetate hydrate, the filtered mother liquor enters into the liquid phase separator for static layering, the upper layer enters into evaporator 1 and is evaporated to obtain the condensate Benzene, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 9.48 kg of solid potassium acetate hydrate; add 25% dilute sulfuric acid (configured by 98% concentrated sulfuric acid) into the pH adjustment kettle to neutralize to pH = 7.5, then the material liquid goes to the DMF recycling system, the component in the pH adjustment kettle is shown in Table 8.

**Table 8 component of the material liquid to DMF recycling system**

| Component | DMF | water | acetic acid |
|---|---|---|---|
| mass content % | 90.32 | 9.6 | 0.06 |

### Embodiment 5

DMF stock solution component is shown in Table 9.

**Table 9 DMF stock solution component**

| Component | DMF | water | acetic acid |
|---|---|---|---|
| mass content % | 86.1 | 3.9 | 10.0 |

Step (1): add 3 m³ DMF stock solution into the neutralization kettle, add 557.51 kg of solid K₂CO₃ (99% purity, commercially available), pH = 8.0 after addition, heat to 70°C, continue to stir and react for 1.5 h at a stirring rate of 300 r/min, input the filtered mother liquor into the mixing kettle.

Step (2): add 6 m³ toluene (99% purity, commercially available) into the mixing kettle, stir rapidly for 1 h at a stirring rate of 350 r/min, filter in the solid-liquid separator to obtain 568.2 kg of solid potassium acetate hydrate, the filtered mother liquor enters into the liquid phase separator for static layering, the upper layer enters into evaporator 1 and is evaporated to obtain the condensate toluene, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 11.22 kg of solid potassium acetate hydrate; add 50% dilute phosphoric acid (configured by 98% concentrated phosphoric acid) into the pH adjustment kettle to neutralize to pH = 7.0, then the material liquid goes to the DMF recycling system, the component in the pH adjustment kettle is shown in Table 10.

**Table 10 component of the material liquid to DMF recycling system**

| Component | DMF | water | acetic acid |
|---|---|---|---|
| mass content % | 95.15 | 4.83 | 0.02 |

### Embodiment 6

DMF stock solution component is shown in Table 11.

**Table 11 DMF stock solution component**

| Component | DMF | water | acetic acid |
|---|---|---|---|
| mass content % | 87.07 | 4.01 | 8.92 |

Step (1): add 2 m³ DMF stock solution into the neutralization kettle, add 629.33 kg of solid KHCOs (99% purity, commercially available), pH = 8.1 after addition, heat to 70°C, continue to stir and react for 1.5 h at a stirring rate of 300 r/min, input the filtered mother liquor into the mixing kettle.

Step (2): add 5 m³ xylene (99% purity, commercially available) into the mixing kettle, stir rapidly for 1 h at a stirring rate of 400 r/min, filter in the solid-liquid separator to obtain 600.51 kg of solid potassium acetate hydrate, the filtered mother liquor enters into the liquid phase separator for static layering, the upper layer enters into evaporator 1 and is evaporated to obtain the condensate xylene, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 16.15 kg of solid potassium acetate hydrate; add 15% dilute sulfuric acid/phosphoric acid into the pH adjustment kettle to neutralize to pH = 7.5, then the material liquid goes to the DMF recycling system, the component in the pH adjustment kettle is shown in Table 12.

**Table 12 component of the material liquid to DMF recycling system**

| Component | DMF | water | acetic acid |
|---|---|---|---|
| mass content % | 95.23 | 4.76 | 0.01 |

### Embodiment 7

DMF stock solution component is shown in Table 13.

**Table 13 DMF stock solution component**

| Component | DMF | water | acetic acid |
|---|---|---|---|
| mass content % | 74.92 | 15.07 | 10.01 |

Step (1): add 1 m³ DMF stock solution into the neutralization kettle, add 123.41 kg of solid Ca(OH)₂ (99% purity, commercially available), pH = 9.5 after addition, heat to 65°C, continue to stir and react for 45 min at a stirring rate of 250 r/min, input the filtered mother liquor into the mixing kettle.

Step (2): add 3 m³ toluene (99% purity, commercially available) into the mixing kettle, stir rapidly for 1 h at a stirring rate of 400 r/min, filter in the solid-liquid separator to obtain 288.42 kg of solid calcium acetate monohydrate, the filtered mother liquor enters into the liquid phase separator for static layering, the upper layer enters into evaporator 1 and is evaporated to obtain the condensate toluene, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 5.11 kg of solid calcium acetate monohydrate; add 30% dilute phosphoric acid (configured by 98% concentrated phosphoric acid) into the pH adjustment kettle to neutralize to pH = 8.0, then the material liquid goes to the DMF recycling system, the component in the pH adjustment kettle is shown in Table 14.

**Table 14 component of the material liquid to DMF recycling system**

| Component | DMF | water | acetic acid |
|---|---|---|---|
| mass content % | 82.78 | 17.21 | 0.01 |

### Embodiment 8

DMF stock solution component is shown in Table 15.

**Table 15 DMF stock solution component**

| Component | DMF | water | acetic acid |
|---|---|---|---|
| mass content % | 84.13 | 7.58 | 8.29 |

Step (1): add 2.5 m³ DMF stock solution into the neutralization kettle, add 383.38 kg of solid Ca(OH)₂ (99% purity, commercially available), pH = 11 after addition, heat to 50°C, continue to stir and react for 50 min at a stirring rate of 300 r/min, input the filtered mother liquor into the mixing kettle.

Step (2): add 5 m³ ether (99% purity, commercially available) into the mixing kettle, stir rapidly for 50 min at a stirring rate of 400 r/min, filter in the solid-liquid separator to obtain 570.25 kg of solid calcium acetate monohydrate, the filtered mother liquor enters into the liquid phase separator for static layering, the upper layer enters into evaporator 1 and is evaporated to obtain the condensate ether, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 37.75 kg of solid calcium acetate monohydrate; add 30% dilute sulfuric acid (configured by 98% concentrated sulfuric acid) into the pH adjustment kettle to neutralize to pH = 8.0, then the material liquid goes to the DMF recycling system, the component in the pH adjustment kettle is shown in Table 16.

**Table 16 component of the material liquid to DMF recycling system**

| Component | DMF | water | acetic acid |
|---|---|---|---|
| mass content % | 90.62 | 9.37 | 0.01 |

### Embodiment 9

DMF stock solution component is shown in Table 17.

**Table 17 DMF stock solution component**

| Component | DMF | water | acetic acid |
|---|---|---|---|
| mass content % | 92.07 | 5.56 | 2.37 |

Step (1): add 800L DMF stock solution into the neutralization kettle, add 163.04 kg of solid Na₂CO₃ (99% purity, commercially available), add 51.2 kg of solid NaHCOs (99% purity, commercially available), pH = 9.1 after addition, heat to 50°C, continue to stir and react for 30 min at a stirring rate of 100 r/min, input the filtered mother liquor into the mixing kettle.

Step (2): add 400L toluene (99% purity, commercially available) into the mixing kettle, stir rapidly for 30 min at a stirring rate of 200 r/min, filter in the solid-liquid separator to obtain 388.48 kg of solid sodium acetate trihydrate, the filtered mother liquor enters into the liquid phase separator for static layering, the upper layer enters into evaporator 1 and is evaporated to obtain the condensate toluene, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 17.12 kg of solid sodium acetate trihydrate; add 45% dilute phosphoric acid (configured by 98% concentrated phosphoric acid) into the pH adjustment kettle to neutralize to pH = 8.0, then the material liquid goes to the DMF recycling system, the component in the pH adjustment kettle is shown in Table 18.

**Table 18 component of the material liquid to DMF recycling system**

| Component | DMF | water | acetic acid |
|---|---|---|---|
| mass content % | 93.61 | 6.31 | 0.08 |

### Embodiment 10

DMF stock solution component is shown in Table 19.

**Table 19 DMF stock solution component**

| Component | DMF | water | acetic acid |
|---|---|---|---|
| mass content % | 92.88 | 5.23 | 1.89 |

Step (1): add 500L DMF stock solution into the neutralization kettle, add 4.18 kg of solid K₂CO₃ (99% purity, commercially available), add 5.13 kg of solid KHCO₃ (99% purity, commercially available), pH = 7.8 after addition, heat to 40°C, continue to stir and react for 30 min at a stirring rate of 150 r/min, input the filtered mother liquor into the mixing kettle.

Step (2): add 250L anisole (99% purity, commercially available) into the mixing kettle, stir rapidly for 15 min at a stirring rate of 200 r/min, filter in the solid-liquid separator to obtain 14.7 kg of solid potassium acetate hydrate, the filtered mother liquor enters into the liquid phase separator for static layering, the upper layer enters into evaporator 1 and is evaporated to obtain the condensate anisole, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 0.48 kg of solid potassium acetate hydrate; add 3% dilute phosphoric acid (configured by 98% concentrated phosphoric acid) into the pH adjustment kettle to neutralize to pH = 7.0, then the material liquid goes to the DMF recycling system, the component in the pH adjustment kettle is shown in Table 20.

**Table 20 component of the material liquid to DMF recycling system**

| Component | DMF | water | acetic acid |
|---|---|---|---|
| mass content % | 93.97 | 5.98 | 0.05 |

### Embodiment 11

DMF stock solution component is shown in Table 21.

**Table 21 DMF stock solution component**

| Component | DMF | water | acetic acid |
|---|---|---|---|
| mass content % | 84.78 | 9.24 | 5.98 |

Step (1): add 5m³ DMF stock solution into the neutralization kettle, add 553.25 kg of solid Ca(OH)₂ (99% purity, commercially available), pH = 10.5 after addition, heat to 55°C, continue to stir and react for 45 min at a stirring rate of 250 r/min, input the filtered mother liquor into the mixing kettle.

Step (2): add 2.5 m³ xylene (99% purity, commercially available) into the mixing kettle, stir rapidly for 30 min at a stirring rate of 200 r/min, filter in the solid-liquid separator to obtain 869.51 kg of solid calcium acetate monohydrate, the filtered mother liquor enters into the liquid phase separator for static layering, the upper layer enters into evaporator 1 and is evaporated to obtain the condensate xylene, which is returned to the mixing kettle to continue to participate in the anti-solvent extraction and crystallization process, is evaporated to obtain the concentrated solution entering into the pH adjustment kettle; the lower layer enters into the evaporator 2 and is evaporated to obtain the condensate entering into the pH adjustment kettle, so as to obtain 5.07 kg of solid sodium acetate trihydrate; add 15% dilute sulfuric acid/phosphoric acid into the pH adjustment kettle to neutralize to pH = 6.0, then the material liquid goes to the DMF recycling system, the component in the pH adjustment kettle is shown in Table 22.

**Table 22 component of the material liquid to DMF recycling system**

| Component | DMF | water | acetic acid |
|---|---|---|---|
| mass content% | 89.72 | 10.25 | 0.03 |

In summary, the DMF recycling method of this application converts the acetic acid in the DMF stock solution into acetate by first adding an alkaline neutralizer, and then achieves the removal of acetic acid by adding an anti-solvent that is insoluble with acetate for extraction and crystallization, and then obtains a crude DMF solution almost free of acetic acid by layering and evaporation of mother liquor, the crude DMF solution is used for DMF recycling to avoid reaction with metal equipment due to the presence of acetic acid in the raw material, which will not corrode the equipment and generate tetramethylurea mixed with DMF. Thus, the DMF recycling method of the present application can maintain the safety of the equipment and avoid corrosion, but also improve the recycling purity of DMF and reduce the content of tetramethylurea impurities in the recycled DMF. In addition, in the preferred embodiment of the present application, the precipitated acetate hydrate can also be used as a carbon source for the biochemical system of the municipal wastewater station for feeding bacteria, thus making secondary use of the product and further improving the economic benefits of the present recycling method.

The foregoing is only the specific embodiment of the present invention, and under the above-mentioned teaching of the present invention, those skilled in the art can perform other improvements or deformations on the basis of the above-described embodiments. Those skilled in the art should understand that the above specific description is only to better explain the object of the present invention, and the protection scope of the present invention should be determined by the protection scope of the claims.

## Claims

1. A DMF recycling method, for recycling DMF, acetic acid and water comprised in DMF stock solution used, is **characterized in that**, the method comprises the following steps:
neutralization step: adding alkaline neutralizer to the DMF stock solution, controlling pH value of a mixture within a preset range, and performing a neutralization reaction to convert acetic acid in the DMF stock solution into acetate;
extraction and crystallization step: adding an anti-solvent that is insoluble with the acetate to the neutralized solution, mixing and stirring to extract DMF by the anti-solvent, thereby causing an acetate hydrate to crystallize and precipitate, and filtering the solid to obtain mother liquor containing DMF;
layering step: standing and layering the mother liquor containing DMF to obtain an upper layer liquid phase and a lower layer liquid phase;
evaporation step: the upper layer liquid phase and the lower layer liquid phase are evaporated and concentrated separately; a condensate obtained by evaporation of the upper layer liquid phase is recycled into the anti-solvent, a concentrated solution obtained by evaporation of the upper layer liquid phase and a condensate obtained by evaporation of the lower layer liquid phase are mixed to obtain a crude DMF solution for recycling of DMF.

2. The DMF recycling method according to claim 1, is **characterized in that**, in the DMF stock solution: DMF mass content is 75% to 95%, water mass content is 4% to 15%, and acetic acid mass content is 1% to 10% .

3. The DMF recycling method according to claim 1, is **characterized in that**, the alkaline neutralizer is liquid alkali or solid alkali; the liquid alkali is one of NaOH or KOH aqueous solution, and mass concentration is 1% to 50%; the solid alkali is one of NaHCOs, Na₂CO₃, KHCOs, K₂CO₃ or Ca(OH)2, or a mixture of NaHCOs and Na₂CO₃, or a mixture of KHCOs and K₂CO₃, preferably Ca(OH)₂.

4. The DMF recycling method according to claim 3, is **characterized in that**, the amount of solute in the liquid alkali is 0.8 time to 1 time of the amount of substance of acetic acid, reaction temperature is room temperature, reaction time is 5 min to 15 min, and stirring rate is 100 r/min to 300 r/min.

5. The DMF recycling method according to claim 3, is **characterized in that**, the amount of the solid alkali is 1 to 3 times of the amount of substance of acetic acid, reaction temperature is 40°C to 70°C, reaction time is 0.5h to 1.5h, and stirring rate is 200 r/min to 400 r/min.

6. The DMF recycling method according to claim 1, is **characterized in that**, in the neutralization step, the pH of the mixture is controlled from 7 to 11 by adding an alkaline neutralizing agent.

7. The DMF recycling method according to claim 1, is **characterized in that**, the anti-solvent is one of ether, anisole, isopropyl ether, benzene, toluene and xylene; preferably the anti-solvent is toluene.

8. The DMF recycling method according to claim 1, is **characterized in that**, the anti-solvent is added in the amount of: volume ratio of DMF stock solution: anti-solvent = 1: 0.5 to 5; preferably: volume ratio of DMF stock solution: anti-solvent = 1: 1 to 3.

9. The DMF recycling method according to claim 1, is **characterized in that**, in the extraction and crystallization step, the temperature of anti-solvent extraction and crystallization process is room temperature, the stirring rate is 200 r/min to 400 r/min, and the stirring time is 15 min to 60min.

10. The DMF recycling method according to claim 1, is **characterized in that**, the method also comprises the step: adjustment step: adding an acidic regulator to the crude DMF solution to adjust the pH of the crude DMF solution to 6-8, preferably 7, and the adjusted crude DMF solution is used to recycle DMF.

11. The DMF recycling method according to claim 10, is **characterized in that**, the acid regulator used in the adjustment step is inorganic acid, preferably a mixture of one or more of sulfuric acid, phosphoric acid solution, with a mass concentration of 5% to 50%.

12. The DMF recycling method according to claim 1, is **characterized in that**, the method also comprises the step: secondary recycling step: recycling acetate hydrate solid precipitated in the extraction and crystallization step and/or acetate hydrate solid obtained by evaporation of the lower layer liquid phase in the evaporation step.

## Patentansprüche

1. DMF-Recyclingverfahren zum Recyceln von DMF, Essigsäure und Wasser, die in verwendeter DMF-Stammlösung umfasst sind, **dadurch gekennzeichnet, dass** das Verfahren die folgenden Schritte umfasst:
Neutralisationsschritt: Zugeben von alkalischem Neutralisator zur DMF-Stammlösung, Steuern des pH-Werts eines Gemisches innerhalb eines voreingestellten Bereichs, und Durchführen einer Neutralisationsreaktion, um Essigsäure in der DMF-Stammlösung in Acetat umzuwandeln;
Extraktions- und Kristallisationsschritt: Zugeben eines Antilösungsmittels, das mit dem Acetat unlöslich ist, zu der neutralisierten Lösung, Mischen und Rühren, um DMF durch das Antilösungsmittel zu extrahieren, dadurch Bewirken, dass ein Acetathydrat kristallisiert und ausfällt, und Filtern des Feststoffs, um DMF-haltige Mutterlauge zu erhalten;
Schichtungsschritt: Stehen- und Schichtenlassen der DMFhaltigen Mutterlauge, um eine flüssige Phase der oberen Schicht und eine flüssige Phase der unteren Schicht zu erhalten;
Verdampfungsschritt: die flüssige Phase der oberen Schicht und die flüssige Phase der unteren Schicht werden getrennt verdampft und konzentriert; ein Kondensat, das durch Verdampfung der flüssigen Phase der oberen Schicht erhalten wird, wird zu dem Antilösungsmittel recycelt, eine konzentrierte Lösung, die durch Verdampfung der flüssigen Phase der oberen Schicht erhalten wird, und ein Kondensat, das durch Verdampfung der flüssigen Phase der unteren Schicht erhalten wird, werden gemischt, um eine rohe DMF-Lösung zum Recyceln von DMF zu erhalten.

2. DMF-Recyclingverfahren nach Anspruch 1, **dadurch gekennzeichnet, dass** in der DMF-Stammlösung: der Massenanteil von DMF 75 % bis 95 % beträgt, der Massenanteil von Wasser 4 % bis 15 % beträgt, und der Massenanteil von Essigsäure 1 % bis 10 % beträgt.

3. DMF-Recyclingverfahren nach Anspruch 1, **dadurch gekennzeichnet, dass** der alkalische Neutralisator flüssiges Alkali oder festes Alkali ist; das flüssige Alkali eine von wässriger NaOH- oder KOH-Lösung ist, und die Massenkonzentration 1 % bis 50 % beträgt; das feste Alkali eines von NaHCOs, Na₂CO₃, KHCOs, K₂CO₃ oder Ca(OH)₂, oder ein Gemisch von NaHCOs und Na₂CO₃, oder ein Gemisch von KHCO₃ und K₂CO₃, bevorzugt Ca(OH)₂ ist.

4. DMF-Recyclingverfahren nach Anspruch 3, **dadurch gekennzeichnet, dass** die Menge an gelöstem Stoff im flüssigen Alkali das 0,8-Fache bis 1-Fache der Stoffmenge von Essigsäure beträgt, die Reaktionstemperatur Raumtemperatur ist, die Reaktionszeit 5 Min. bis 15 Min. beträgt und die Rührgeschwindigkeit 100 U/min bis 300 U/min beträgt.

5. DMF-Recyclingverfahren nach Anspruch 3, **dadurch gekennzeichnet, dass** die Menge des festen Alkalis das 1-bis 3-Fache der Stoffmenge von Essigsäure beträgt, die Reaktionstemperatur 40 °C bis 70 °C beträgt, die Reaktionszeit 0,5 Std. bis 1,5 Std. beträgt und die Rührgeschwindigkeit 200 U/min bis 400 U/min beträgt.

6. DMF-Recyclingverfahren nach Anspruch 1, **dadurch gekennzeichnet, dass** im Neutralisationsschritt der pH-Wert des Gemisches durch Zugeben eines alkalischen Neutralisationsmittels von 7 bis 11 gesteuert wird.

7. DMF-Recyclingverfahren nach Anspruch 1, **dadurch gekennzeichnet, dass** das Antilösungsmittel eines von Ether, Anisol, Isopropylether, Benzol, Toluol und Xylol ist; bevorzugt das Antilösungsmittel Toluol ist.

8. DMF-Recyclingverfahren nach Anspruch 1, **dadurch gekennzeichnet, dass** das Antilösungsmittel in folgender Menge zugegeben wird: Volumenverhältnis DMF-Stammlösung : Antilösungsmittel = 1 : 0,5 bis 5; bevorzugt: Volumenverhältnis DMF-Stammlösung : Antilösungsmittel = 1 : 1 bis 3.

9. DMF-Recyclingverfahren nach Anspruch 1, **dadurch gekennzeichnet, dass** im Extraktions- und Kristallisationsschritt die Temperatur des Antilösungsmittel-Extraktions- und Kristallisationsprozesses Raumtemperatur ist, die Rührgeschwindigkeit 200 U/min bis 400 U/min beträgt und die Rührzeit 15 Min. bis 60 Min. beträgt.

10. DMF-Recyclingverfahren nach Anspruch 1, **dadurch gekennzeichnet, dass** das Verfahren auch den folgenden Schritt umfasst: Einstellungsschritt: Zugeben eines sauren Regulators zur rohen DMF-Lösung, um den pH-Wert der rohen DMF-Lösung auf 6-8, bevorzugt 7, einzustellen, und die eingestellte rohe DMF-Lösung verwendet wird, um DMF zu recyceln.

11. DMF-Recyclingverfahren nach Anspruch 10, **dadurch gekennzeichnet, dass** der im Einstellungsschritt verwendete Säureregulator eine anorganische Säure ist, bevorzugt ein Gemisch aus einer oder mehreren von Schwefelsäure, Phosphorsäurelösung, mit einer Massenkonzentration von 5 % bis 50 %.

12. DMF-Recyclingverfahren nach Anspruch 1, **dadurch gekennzeichnet, dass** das Verfahren auch den folgenden Schritt umfasst: sekundärer Recyclingschritt: Recyceln von Acetathydrat-Feststoff, der im Extraktions- und Kristallisationsschritt ausgefällt wurde, und/oder Acetathydrat-Feststoff, der durch Verdampfung der flüssigen Phase der unteren Schicht im Verdampfungsschritt erhalten wurde.

## Revendications

1. Procédé de recyclage de DMF, pour recycler le DMF, l'acide acétique et l'eau contenus dans une solution mère de DMF utilisée, **caractérisé en ce que** le procédé comprend les étapes suivantes :
étape de neutralisation : ajouter un neutralisant alcalin à la solution mère de DMF, ajuster la valeur du pH d'un mélange dans une plage prédéfinie, et réaliser une réaction de neutralisation pour convertir l'acide acétique dans la solution mère de DMF en acétate ;
étape d'extraction et de cristallisation : ajouter un anti-solvant qui est insoluble dans l'acétate à la solution neutralisée, mélanger et agiter pour extraire le DMF par l'anti-solvant, pour ainsi provoquer la cristallisation et la précipitation d'un acétate hydraté, et filtrer le solide pour obtenir une liqueur mère contenant du DMF ;
étape d'organisation en phases : laisser reposer et s'organiser en phases la liqueur mère contenant du DMF pour obtenir une phase liquide supérieure et une phase liquide inférieure ;
étape d'évaporation : la phase liquide supérieure et la phase liquide inférieure sont évaporées et concentrées séparément ; un condensat obtenu par évaporation de la phase liquide supérieure est recyclé dans l'anti-solvant, une solution concentrée obtenue par évaporation de la phase liquide supérieure et un condensat obtenu par évaporation de la phase liquide inférieure sont mélangés pour obtenir une solution de DMF brute pour le recyclage du DMF.

2. Procédé de recyclage de DMF selon la revendication 1, **caractérisé en ce que**, dans la solution mère de DMF : la teneur massique en DMF est de 75 % à 95 %, la teneur massique en eau est de 4 % à 15 %, et la teneur massique en acide acétique est de 1 % à 10 %.

3. Procédé de recyclage de DMF selon la revendication 1, **caractérisé en ce que** le neutralisant alcalin est un alcalin liquide ou un alcalin solide ; l'alcalin liquide est une solution aqueuse de NaOH ou de KOH, et la concentration massique est de 1 % à 50 % ; l'alcalin solide est l'un parmi NaHCOs, Na₂CO₃, KHCO₃, K₂CO₃ ou Ca(OH)2, ou un mélange de NaHCOs et de Na₂CO₃, ou un mélange de KHCO₃ et de K₂CO₃, de préférence Ca(OH)₂.

4. Procédé de recyclage de DMF selon la revendication 3, **caractérisé en ce que** la quantité de soluté dans l'alcalin liquide est de 0,8 fois à 1 fois la quantité de substance d'acide acétique, la température de réaction est la température ambiante, le temps de réaction est de 5 min à 15 min, et la vitesse d'agitation est de 100 tr/min à 300 tr/min.

5. Procédé de recyclage de DMF selon la revendication 3, **caractérisé en ce que** la quantité d'alcalin solide est de 1 à 3 fois la quantité de substance d'acide acétique, la température de réaction est de 40 °C à 70 °C, le temps de réaction est de 0,5 h à 1,5 h, et la vitesse d'agitation est de 200 tr/min à 400 tr/min.

6. Procédé de recyclage de DMF selon la revendication 1, **caractérisé en ce que**, dans l'étape de neutralisation, le pH du mélange est ajusté entre 7 et 11 par ajout d'un agent neutralisant alcalin.

7. Procédé de recyclage de DMF selon la revendication 1, **caractérisé en ce que** l'anti-solvant est l'un parmi l'éther, l'anisole, l'isopropyléther, le benzène, le toluène et le xylène ; de préférence, l'anti-solvant est le toluène.

8. Procédé de recyclage de DMF selon la revendication 1, **caractérisé en ce que** l'anti-solvant est ajouté dans la quantité suivante : rapport volumique solution mère de DMF:anti-solvant = 1:0,5 à 5 ; de préférence : rapport volumique solution mère de DMF:anti-solvant = 1:1 à 3.

9. Procédé de recyclage de DMF selon la revendication 1, **caractérisé en ce que**, dans l'étape d'extraction et de cristallisation, la température du processus d'extraction et de cristallisation par anti-solvant est la température ambiante, la vitesse d'agitation est de 200 tr/min à 400 tr/min, et le temps d'agitation est de 15 min à 60 min.

10. Procédé de recyclage de DMF selon la revendication 1, **caractérisé en ce que** le procédé comprend également l'étape : étape d'ajustement : ajouter un régulateur acide à la solution de DMF brute pour ajuster le pH de la solution de DMF brute à 6-8, de préférence 7, et la solution de DMF brut ajustée est utilisée pour recycler le DMF.

11. Procédé de recyclage de DMF selon la revendication 10, **caractérisé en ce que** le régulateur d'acide utilisé dans l'étape d'ajustement est un acide inorganique, de préférence un mélange d'un ou de plusieurs parmi l'acide sulfurique, une solution d'acide phosphorique, avec une concentration massique de 5 % à 50 %.

12. Procédé de recyclage de DMF selon la revendication 1, **caractérisé en ce que** le procédé comprend également l'étape : étape de recyclage secondaire : recycler l'acétate hydraté solide précipité dans l'étape d'extraction et de cristallisation et/ou l'acétate hydraté solide obtenu par évaporation de la phase liquide inférieure dans l'étape d'évaporation.
